# EUROPEAN PATENT APPLICATION

(11) **EP 4 279 097 A1**
(43) Date of publication of application: **22.11.2023**
(21) Application number: 22174090.5
(22) Date of filing: 18.05.2022
(51) Int. Cl.: A61M 5/00, A61M 5/32, A61M 5/50, B65D 75/66, G09F 3/00

(54) **TAMPER PROOF LABEL**

(71) Applicant: Terumo Europe NV, 3001 Leuven (BE)
(72) Inventor: CASTELEYN, Pieter Nico Jan, 3020 Herent (BE); VALKENAERS, Hans, 2221 Booischot (BE); DANIELS, Ludo, 3700 Tongeren (BE); FRIPON, Christian, 3053 Haasrode (BE)
(74) Representative: V.O.

(57) **Abstract**

The present disclosure pertains to a packaged needle assembly product (100) comprising: a hard-shell packaging product comprising a case (2) and a cap (3) arranged in an assembled configuration forming an enclosure for the needle assembly , and an adhesive label (13) affixed to an outer face of the enclosure, wherein the label extends across at least a portion of a circumferential gap (g) between the cap and the case thereby sealing the hard-shell packaging product (1). The label includes a perforated section that comprises a plurality of perforation lines 51 that cross the gap under an angle from a first end that terminates at or beyond the edge of the cap to a second end that terminates at or beyond the edge of the case across the gap.

## Description

### TECHNICAL FIELD AND BACKGROUND

The invention generally relates to medical equipment and, more specifically, to tamper proof label for a hard case packaging product for sterile packaging of a needle assembly for use in medical injection equipment. Safety needle assemblies are widely known and used. Typically, a safety needle assembly comprises a hub having a proximal end for connecting with a syringe. Prior to the use the assembly is stored in a package under sterile conditions.

Blister packaging solutions are well known. Upon use the user, e.g. a paramedical person partially opens the blister to make the hub free, and then holds the assembly at the packaging to connect the hub to the syringe to avoid contamination or a possible sterility breach of the needle assembly. Then, the user further removes the packaging to make the safety needle assembly free. This process is rather complex and can be prone to user error.

Hard case packaging products that include a cap and a case can be used as an alternative packaging solution which is generally more compatible with high speed automated packaging processes than blister packages.

However, the comparatively more rigid enclosure can be more challenging in terms of providing a reliable indicator that reassures an end user that the package has not been tampered with or opened prior to an end use. For example, without specific measures re-closing an opened package may be attempted by repositioning the cap onto the case.

As such there remains a need to provide tamper proof packaging solutions to reassure an end user of product sterility. In particulate there remains a need for a solution that irreversible provides highly visible indications of an opening event while retaining compatibility with automated machine packaging processes.

### SUMMARY

The present disclosure aims to mitigate one or more of the above disadvantages. Aspects of the present disclosure related to a packaged needle assembly product. In particular a packaged needle assembly product comprising: a hard-shell packaging product that comprises a case and a cap that are mutually arranged in an assembled configuration to form an enclosure for the needle assembly. To seal, close, the enclosure the packaged needle assembly product further comprises an adhesive label. The adhesive label is affixed to an outer face of the enclosure, wherein the label extends across at least a portion of a gap the cap and the case. Typically a circumferential between an edge of the cap and an edge of the case thereby.

The label includes a perforated section that comprises a plurality of perforation lines. The perforation lines are arranged under an angle relative to the gap. The perforation extend across the gap from a first end that terminates at or beyond a first boundary of the gap, e.g. the edge of the cap, to a second end that terminates at or beyond a second boundary of the gap, e.g. the edge of the case, across the gap.

As will be detailed herein below the label provides a tamper proofing that reassures an end user of a quality of the packed product. The plurality of perforation lines was found to provide an effective visual indication of a prior opening event or attempt to re-close the packaging, by a presence of tear marks, e.g. raffles.

The performed lines are preferably arranged under an angle θ in a range of 20 to 40 degrees with regard to the circumferential gap, more preferably between 25 and 35 degrees, most preferably about 30 degrees, e.g. 28-32 degrees. An angle of 20-40 degrees as found to constitute a good range for tear propagation, and thus formation of highly visible raffles, upon opening. Accordingly, the inclination of the perforation lines (180°-θ) as defined by the positive angle less than 180 degrees as measured counterclockwise from the gap to perforation line is preferably within 140-160°, more preferably between 145 and 155 degrees, most preferably about 150 degrees, e.g. 148-152 degrees. As will be detailed herein below applicant found that the specified perforation present an optimum in terms of optimizing visibility of tear marks, stability of the label prese prior to and during assembly onto the packaging product, and in providing an optimized user experience as to ranges as force required to access the packaged product.

The label and perforated section is preferably of at least 50% of the length of the perimeter of the enclosure at the gap, preferably at least 80%. The larger the label the more secure the cap is affixed to the case and the larger the potential real-estate available on the label for product information and/or user instructions. The label may be longer than a perimeter of the enclosure so that the label wraps around onto itself.

The perforated lines are preferably separated from adjacent ones by a distance of ≤ 1mm, preferably in a range of 0.7 to 0.9 mm. Larger separations were found to require increasingly large tear forces and result in less predictable quality of a formed tear pattern. Smaller separations were found to result increasingly an overall fragility of the label as a whole. prior to or during (machine)application onto the cap and case.

The length of the perforations and accordingly the width of the perforated section in a direction orthogonal to a split line between cap and case is preferably such that the lines cover manufacturing tolerances.

Preferably, the perforations extend across the gap by a distance at least 0.1 mm at both ends. Most preferably the perforations extend across the gap at least one end by at least 0.5 mm. The longer the overlap the more visible the tear marks. To mitigate weakening the label the perforated section preferably does not extend beyond 50% of an overall height of the label. Machine handling of a label having a non-perforated boundary of about 1 mm (top, bottom and sides) was confirmed.

Preferably, all of the perforation lines are arranged essentially parallelly within a single file. Other arrangements, including perforations in two or more files, arrangements with crossing perforations, and/or arrangement with perforations extending under differently inclination are less preferred. Additional perforations and/or other configurations were found to reduce tear pattern visibility and/or reduce the label's structural integrity increasing a potential of pre-mature tear or rupture, in response to a application of a force normal to the label, e.g. during roll-on application.

In a preferred embodiment, the case and the cap are configured to engage slidingly to the assembled configuration, and wherein the case and the cap comprise circumferentially overlapping sidewall sections. Advantageously, the hard-shell packaging product can comprise one or more radial channel that extends from the gap along a longitudinal direction between the overlapping sidewall sections so as to provide a gas passage P to an interior of the enclosure.

The channel can at least in part be defined by one or more protrusion that extends outwardly from at least one of an inner face of a circumferentially overlapping wall section of the cap towards a corresponding overlapping wall section of the case, and an exterior face to the circumferentially overlapping wall section of the case towards a corresponding wall section of the cap.

Further or additional aspects related t: an adhesive label for sealing a hard-shell packaging product; and to a roll comprising a backing supporting a plurality of the adhesive labels. In particular to an adhesive label for sealing a hard-shell packaging product, e.g. as disclosed herein, that comprises a case and a cap configured to, in an assembled configuration, form an enclosure for a needle assembly, wherein the label is configured to adhere to an outer face of the enclosure and dimensioned to extend across at least a portion of a circumferential gap between an edge of the cap and an edge of the case thereby fixing the case to the cap, and wherein the label includes a perforated section comprising a plurality of perforation lines arranged to, when adhered, cross the gap under an angle from a first end that terminates at or beyond the edge of the cap to a second end that terminates at or beyond the edge of the case across the gap.

### BRIEF DESCRIPTION OF DRAWINGS

These and other features, aspects, and advantages of the apparatus, systems and methods of the present disclosure will become better understood from the following description, appended claims, and accompanying drawing wherein:
FIG 1A depicts a packaged needle assembly;
FIG 1B depicts a hard-shell packaging product in an open condition;
FIG 1C provides a perspective view of a case and a needle assembly;
FIG 2A illustrates an adhesive label;
FIG 2B depicts packaged needle assemblies;
FIG 2C illustrates an adhesive label;
FIG 3A depicts an opened packaged needle assembly product;
FIG 3B depicts a comparative packaged needle assembly product in closed a state (left) and after opening (right); and
FIG 3C illustrates a partial cross-section side view of a hard-shell packaging product.

### DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

As used herein the term needle assembly may be understood as relating to needle assembly comprising a hub having a proximal end for connecting with a syringe extending in a proximal area of the hub; and a needle longitudinally extending from a distal end of the hub in a distal area of the hub.

The needle assembly typically comprises at least a canula (also referred to as needle) that extends from a needle hub for receiving a corresponding counterpart of a syringe. Preferably, the needle assembly is a safely needle assembly comprising a protector that is typically embodied as a longitudinally extending sleeve that can be engaged over the needle, and that at an end there of can be removably connected to the hub, and a sheath that is pivotally connected to the hub. The sheath typically has two side walls connected by a back wall defining a receiving space therebetween for receiving the needle. The sheath is pivotable between a starting position in which the sheath is free from the needle, between a use position in which the sheath is pivoted away from the needle and the needle is exposed, and between a locked position in which the sheath is locked to secure the needle into the receiving space. In the locked position, the sheath protects the needle, after its use, against unintended contact, contamination, needlestick injury etc. In the locked position, the needle is received in the receiving space of the sheath to be enclosed by the sheath, and as such, to cover the needle.

Although not exclusively, the packaging product is of particular benefit for a safety needle assembly, such as a safety needle assembly described in full detail in PCT/EP2021/063205, which is hereby incorporated by reference.

The safety needle assembly preferably comprises a hub having a proximal end for connecting with a syringe extending in a proximal area of the hub; a needle longitudinally extending from a distal end of the hub in a distal area of the hub; a sheath pivotally connected to the hub, the sheath having two side walls connected by a back wall defining a receiving space therebetween for receiving the needle; wherein the sheath is adjustable between a starting position in which the sheath is substantially positioned in the distal area of the hub, between a use position in which the sheath is substantially positioned in the proximal area of the hub, and between a locked position in which the sheath (protector) is locked to secure the needle into the receiving space; wherein in the starting position the sheath is longitudinally extending from the hub, wherein the hub is provided with at least one first cooperating element and the sheath is provided with at least one second cooperating element, wherein, in the locked position of the sheath, the first and the second cooperating elements are engaged to each other for locking the sheath, characterized in that, in the starting position, the first and second cooperating elements are in contact with each other for defining the starting position of the sheath.

By providing the sheath longitudinally extending from the hub in the starting position, the footprint of the safety needle assembly in the starting position, thus for packaging purposes, becomes much less, allowing different types of packaging and/or more efficient packaging and/or transport and/or storage. For example, which such a reduced footprint, a hard case packaging may be considered, allowing automated handling of the packed safety needle assembly. Whereas, automated handling of blister packed assemblies is practically not possible, resulting that the blister packed assemblies need to be collected manually in a box for transport and storage. This limits the speed of assembly lines. By providing the sheath in a longitudinally extended position from the hub in starting position, a more compact arrangement of the safety needle assembly can be obtained, allowing a hard case packaging. Such hard case packaging can be handled automated allowing the speed of the assembly lines to increase, thus allowing a more efficient production, packaging, transport and/or storage of the safety needle assembly. Also, by using a hard cap, the way of working for preparing the needle assembly to use remains unchanged. This is advantageous for the user as he can follow the procedure he is used to. When removing the cap of the hard case, the hub of the needle assembly becomes exposed and can be connected to the syringe without compromising the sterility and avoiding possible contamination. When connected to the syringe, the hard case can be removed from the needle assembly and the sheath can be pivoted to the use position to make the needle with syringe ready for use.

The hard case preferably comprises, or essentially consists of, a medical grade composition, preferably a plastic composition that can be injection molded, such as polypropylene.

The label as described herein typically comprises a substrate and adhesive as known in the field. The adhesive is generally a pressure sensitive adhesive composition. Other adhesives such as temperature sensitive or radiation curable adhesives may be used. Pressure sensitive adhesives advantageously allow machine application, positioning and provision of adhesion pressure (e.g. by rollers).

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

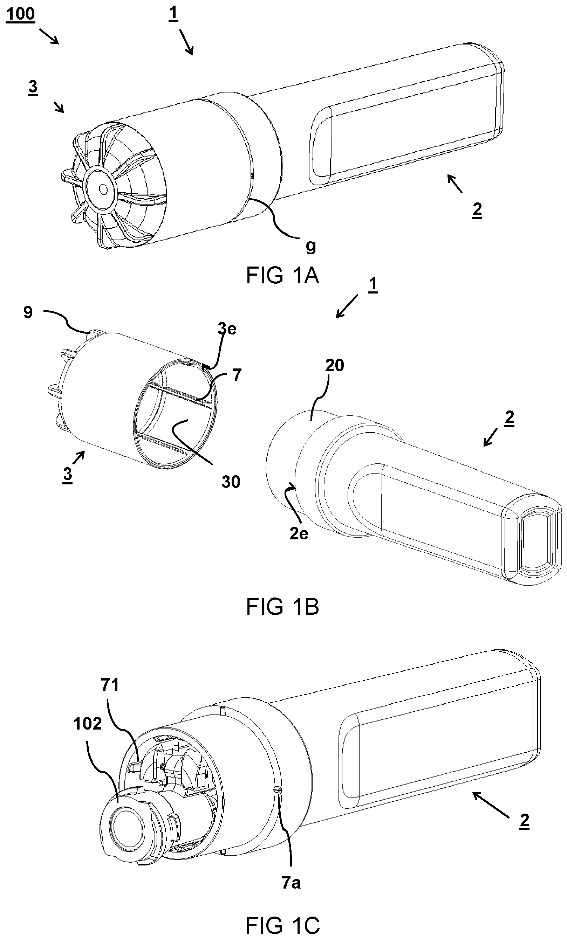

FIG 1A depicts an exemplary embodiment of a packaged needle assembly product 100. The packaged needle assembly product comprises a hard-shell packaging product that forms an enclosure for the needle assembly. The hard-shell packaging product 1 comprises a case 2 and a cap 3 arranged in an assembled configuration forming an enclosure for the needle assembly. A spilt line or gap 'g' is formed between the cap and the case.

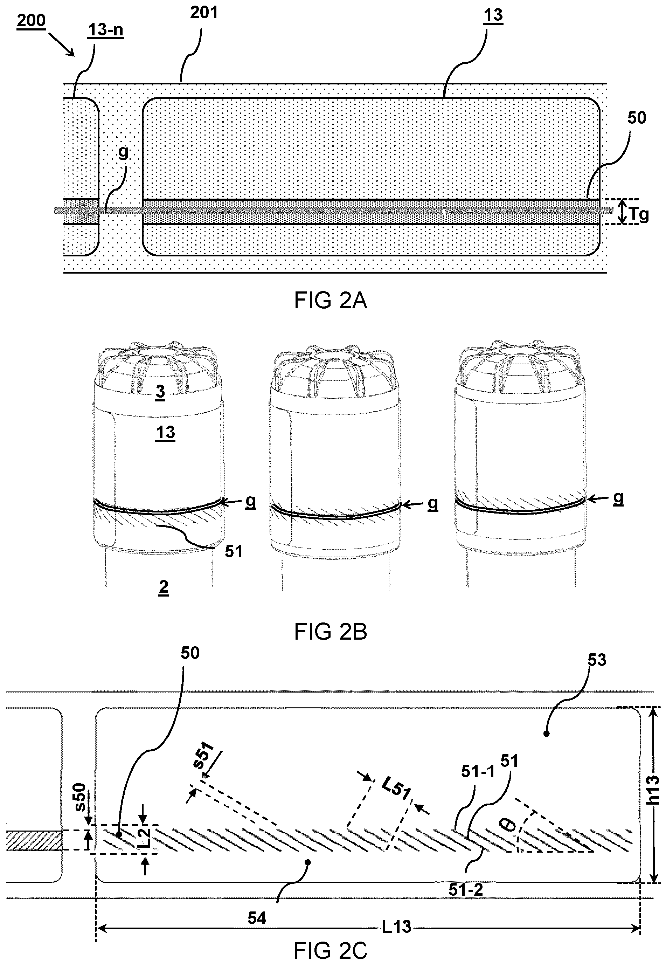

The packaged needle assembly product comprises an adhesive label 13 (see e.g. FIG 2B) that is affixed along an outer face of the enclosure, wherein the label extends across at least a portion of a circumferential gap g between an edge 3e of the cap and an edge 2e of the case thereby fixing the cap to the case and providing a seal onto the hard-shell packaging product 1. Details as to the label will be explained with reference to FIGs 2A, 2B, and 2C.

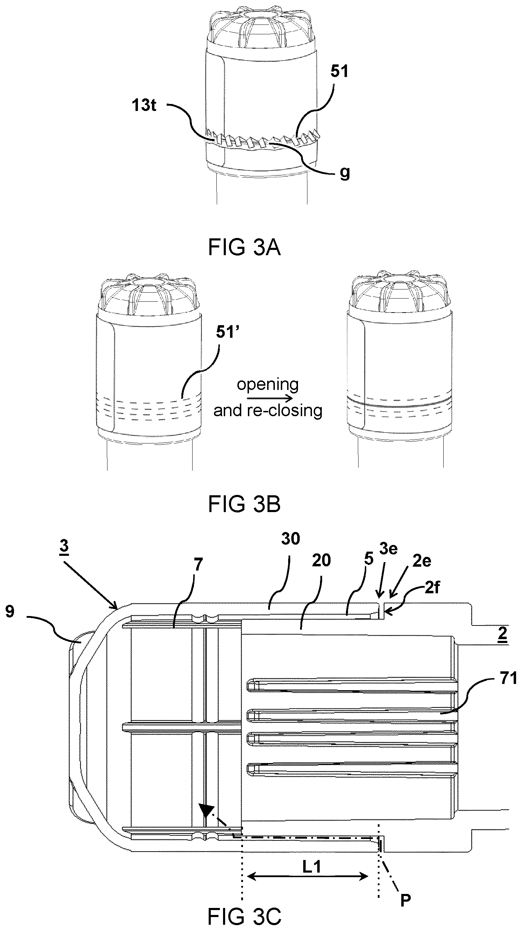

In a preferred embodiment, e.g. as shown in FIG 1B, 1C and 3C, the case 2 and the cap 3 are configured to slidingly engage to the assembled configuration. The case and the cap comprise circumferentially overlapping sidewall sections 20,30.

Advantageously the hard-shell packaging product 1 can comprise one or more radial channel 5 that extends in a longitudinal direction between the overlapping sidewall sections to provide a gas passage P to an interior of the enclosure.

Providing a gas passage to the enclosed volume, advantageously allows sterilizing the packaged product, e.g. the needle assembly. As will be detailed herein below the packing product advantageously allows gas access to the needle assembly for sterilization by a gaseous agent while being enclosed by the cap and the case. Preferably, the channel(s) form the only passage between the interior volume and ambient. By having the channels extend over a distance, e.g. the predesigned engagement distance, between an inner face of the cap and an exterior face of the case (or vice versa) re-contamination of the needle assembly by air-borne infectants is mitigated.

In some embodiments, e.g. as shown, the channel 5 is at least in part defined by one or more protrusion 7 (e.g. rib) that extends outwardly from at least one of an inner face of a circumferentially overlapping wall section of the cap towards a corresponding overlapping wall section of the case, and an exterior face to the circumferentially overlapping wall section of the case towards a corresponding wall section of the cap.

As can be seen in more detail in FIG 1B the spacers can advantageously define a cross-sectional dimension of the channel. The channel length can be understood as being largely defined by an overlap distance between cap and case. The length L1 of the overlap is generally ≥ 2 mm. The longer the overlap the more complex the pathway for ingress of contaminants to ingress. In principle the overlap can be larger. The longer the overlap the more stable the cap can be positioned onto the case. For example, the overlap L1 can be up to about 20 mm, e.g. in a range of 2-20mm or in a range of 3-10 mm. Prevention of re-contamination is believed to relate to the channel providing a stagnant air. An overlap of 2 mm was found to prevent re-contamination. In practice the overlap length L1 is between 5 and 10mm, e.g. about 8mm. A range between 5 and 10 mm was confirmed to be an optimum between compactness and stability of the cap onto the case. The width of the channel (distance between opposing sideways) is preferably as thin as possible while allowing gas passage. Typically, the distance between opposing sideways 'd' is less than 1 mm. In principle the separation can be larger, e.g. up to 2 or even 3 mm or more. The thinner channel the compacter the packaging product can be. Preferably, the separation 'd' is ≤ 0.5. More preferably, d ≤ 0.3 mm, e.g. about 0.2 mm. The wider the channel the larger the access for potential recontamination and the less compact the packed product. A separation of about 0.05 mm was found sufficient.

In a preferred embodiment, e.g. as shown the protrusions form the exclusive contact between opposing sidewall faces of the case and the cap. This limits the area of flush contact between cap and case to essentially point or line contacts as defined by the protrusions.

In some embodiments, e.g. as shown in FIG 1C and 3C one or more further spacers 7a are provided between opposing faces of the cap 3f and the case 2f to assure an axial clearance between cap and case in closed condition, e.g. as indicated in FIGs 1A and 3C.

To prevent internal rotation of the needle assembly 100 within the case 3 one or more inwardly extending ribs 71 or slots can be provided (see e.g. FIG 1C and 3C) that engage with the needle assembly 100, e.g. with hub 102 as indicated).

The case and/or cap parts may comprise a flat portion (e.g. as shown). This flat portion prevents a rolling motion of the product, e.g. along a table top.

To facilitate removal of the cap the cap and/or case can be provided with one or more external members for manual grip, such as the ribs 9 indicated in FIG 1B.

The case and cap as disclosed herein can be of particular benefit for packaging of products in a sterile package that is ready for direct use by an end user. The packaging product advantageously enables sterilizing the product in a package state while providing effective tamper protection by one or more of: members that provide a visual indication of an opening event.

FIG 2A illustrates a section of a backing 201 comprising a plurality of labels 13, 13-n. To facilitate opening and formation of highly visible tear marks 13r the label includes a perforated section 50. The perforated section is dimensioned to overlap with a position of a spilt line or gap g between cap and case. The width of the perforated section 50 is dimensioned to cover overlap with the spilt line included manufacturing tolerances Tg.

FIG 2B provides partial views of packaged needle assembly products 100, e.g. the same product as depicted in FIG 1A, that further comprises the label as disclosed herein. As indicated the perforations overlap splitline or gap g within manufacturing tolerances.

The label fixes spans across the circumferential gap (g) between cap and case (indicated by line). The label fixes the cap to the case. To gain access to the package product a user removes the cap from the case (e.g. by pulling and/or twisting). This causes irreversible damage (tearing) of the label.

As further illustrated in FIG 2C the perforated section 50 comprises a plurality of perforation lines 51. The lines cross the gap under an angle (θ) from a first end 51-1 that terminates at or beyond the edge of the cap to a second end 51-2 that terminates at or beyond the edge of the case across the gap.

The perforated lines are arranged under an angle (θ) with regard to the circumferential gap (g) in a range of 20 to 40 degrees. In a preferred embodiment, e.g. as shown, the angle θ is about 30°.

The length L13 of the label is typically in a range of 80-120% of a perimeter of the hard-shell packaging product at the split line.

The perforated lines 51 are separated from adjacent ones by a distance of ≤ 1mm, preferably in a range of 0.7 to 0.9 mm. Perforations may be suitable made as known in the field, e.g. a rotary drum with blades.

The perforations preferably extend across the gap over a distance s50 by at least 0.1 mm at both ends. Sections 53,54 of the label above and below the perforated section can include one or more data display area for product information and/or user instructions.

The length L13 and height h13 of the label, the width of the perforated section L2, and the length of the individual perforations L51 depend on dimensioning of the cap, case, a width of the gap g and manufacturing tolerances Tg. In some embodiments, e.g. as shown, length L51 can, for example be 3-5 mm. Width L2 of the perforated section can for example be 1-3 mm.

User test studies shown that arranging the perforations under an angle in a range of 20 to 40 degrees, preferably 25-35°, most preferably about 30° consistently resulted in the formation of highly visible tear marks regardless of left-or right handed user operation and regardless of whether the user displaces only the cap, only the case, or both. The preferred right-handed orientation of the perforation lines 51 relative to the split line ties in with a user expectation as to applying an opening force along a counterclockwise directions.

User studies further confirmed that the label as disclosed herein advantageously allows a control over a user-experienced force or torque to open the product..

FIG 3A illustrates an exemplary embodiment of a hard-shell packaging product that has been sealed by a label as disclosed herein after opening of the enclosure, removal of the packed needle assembly, and repositioning of the cap. FIG 3B illustrates a comparative example of an identical hard-shell packaging product before (left) and after opening an re-closing (right), whereby the label was provided with perforation lines 51' that run parallel to a split line between cap and case. As can be seen opening of the product that has been sealed with a label as disclosed herein is clearly visible by presence of a plurality of tear marks 13t (raffled edges), whereas the comparative example results in a clean tear that allows optically re-closing the package after an initial opening due to an absence of clear tear marks.

For the purpose of clarity and a concise description, features are described herein as part of the same or separate embodiments, however, it will be appreciated that the scope of the invention may include embodiments having combinations of all or some of the features described. Of course, it is to be appreciated that any one of the above embodiments may be combined with one or more other embodiments to provide even further improvements in finding and matching designs and advantages.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. Where one claim refers to another claim, this may indicate synergetic advantage achieved by the combination of their respective features. But the mere fact that certain measures are recited in mutually different claims does not indicate that a combination of these measures cannot also be used to advantage. The present embodiments may thus include all working combinations of the claims wherein each claim can in principle refer to any preceding claim unless clearly excluded by context.

## Claims

1. A packaged needle assembly product (100) comprising:
a hard-shell packaging product (1) comprising a case (2) and a cap (3) arranged in an assembled configuration forming an enclosure for the needle assembly , and
an adhesive label (13) affixed to an outer face of the enclosure, wherein the label extends across at least a portion of a circumferential gap (g) between an edge (3e) of the cap and an edge (2e) of the case thereby sealing the hard-shell packaging product (1),
wherein the label includes a perforated section (50) comprising a plurality of perforation lines (51) that cross the gap under an angle (θ) from a first end (51-1) that terminates at or beyond the edge of the cap to a second end (51-2) that terminates at or beyond the edge of the case across the gap.

2. The packaged needle assembly product according to claim 1, wherein the perforated lines are arranged under an angle (θ) with regard to the circumferential gap (g) in a range of 20 to 40 degrees.

3. The packaged needle assembly product according to claim 2, wherein the angle (θ) is between 25 and 35 degrees, preferably about 30 degrees.

4. The packaged needle assembly product according to any of the preceding claims, wherein both the adhesive label (13) and the perforated section (50) extend over a length of at least 80% of the length of the perimeter of the enclosure at the gap.

5. The packaged needle assembly product according to any of the preceding claims, wherein the perforated lines are separated from adjacent ones by a distance of ≤ 1mm, preferably in a range of 0.7 to 0.9 mm.

6. The packaged needle assembly product assembly according to any of the preceding claims, wherein the perforations extend across the gap by at least 0.1 mm at both ends.

7. The packaged needle assembly product according to claim 6, wherein the perforations extend across the gap at least one end by at least 0.5 mm.

8. The packaged needle assembly product according to any of the preceding claims, wherein the label includes a data display area adjacent the performed section.

9. The packaged needle assembly product according to any of the preceding claims, wherein all perforation lines (51) are arranged parallelly within a single file.

10. The packaged needle assembly product according to any of the preceding claims, wherein the case (2) and the cap (3) are configured to slidingly engage to the assembled configuration, and wherein the case and the cap comprise circumferentially overlapping sidewall sections (20,30).

11. The packaged needle assembly product according to claim 10, wherein the hard-shell packaging product (1) comprises one or more radial channel (5) that extends in a longitudinal direction between the overlapping sidewall sections to provide a gas passage (P) to an interior of the enclosure.

12. The packaged needle assembly product according to claim 11, wherein the channel (5) is at least in part defined by one or more protrusion (7) extending outwardly from at least one of an inner face of a circumferentially overlapping wall section of the cap towards a corresponding overlapping wall section of the case, and an exterior face to the circumferentially overlapping wall section of the case towards a corresponding wall section of the cap.

13. An adhesive label (13) for sealing a hard-shell packaging product (1) comprising a case (2) and a cap (3) configured to, in an assembled configuration, form an enclosure for a needle assembly, preferably the hard-shell packaging as defined in any of claims 1-12.
wherein the label is configured to adhere to an outer face of the enclosure and dimensioned to extend across at least a portion of a circumferential gap (g) between an edge of the cap and an edge of the case thereby fixing the case to the cap,
wherein the label includes a perforated section (50) comprising a plurality of perforation lines (51) arranged to, when adhered, cross the gap under an angle (θ) from a first end (51-1) that terminates at or beyond the edge (2e) of the cap to a second end (51-2) that terminates at or beyond the edge (2e) of the case across the gap.

14. A roll (200) comprising a backing (201) supporting a plurality of the adhesive label (13) according to claim 13, or the adhesive label (13) as defined in any of claims 1-12.
